(19) Europäisches Patentamt
European Patent Office
Office européen des brevets

(11) **EP 2 291 230 B1**

(12) **FASCICULE DE BREVET EUROPEEN**

(45) Date de publication et mention de la délivrance du brevet:
**06.08.2014 Bulletin 2014/32**

(51) Int Cl.:
*B01D 53/04* (2006.01)     *B01J 20/20* (2006.01)
*C01B 31/02* (2006.01)     *G01N 33/00* (2006.01)

(21) Numéro de dépôt: **09772639.2**

(22) Date de dépôt: **27.05.2009**

(86) Numéro de dépôt international:
**PCT/FR2009/000612**

(87) Numéro de publication internationale:
**WO 2010/000956 (07.01.2010 Gazette 2010/01)**

(54) **UTILISATION DE NANOMATERIAUX DE CARBONE EN TANT QUE MATERIAU DE FILTRATION IMPERMEABLE A LOZONE**

VERWENDUNG VON CARBON-NANOMATERIALIEN ALS OZON-UNDURCHLÄSSIGES FILTERMATERIAL

USE OF CARBON NANOMATERIALS AS A FILTRATION MATERIAL IMPERVIOUS TO OZONE

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **02.06.2008 FR 0803006**

(43) Date de publication de la demande:
**09.03.2011 Bulletin 2011/10**

(73) Titulaires:
• **Centre National de la Recherche Scientifique 75016 Paris (FR)**
• **Université Blaise Pascal - Clermont-Ferrand II 63006 Clermont-Ferrand Cedex (FR)**

(72) Inventeurs:
• **PAULY, Alain F-63100 Clermont-ferrand (FR)**
• **DUBOIS, Marc F-63000 Clermont-ferrand (FR)**
• **GUERIN, Katia F-63430 Pont Du Chateau (FR)**
• **HAMWI, André F-63000 Clermont-ferrand (FR)**
• **BRUNET, Jérôme F-63200 Riom (FR)**

• **VARENNE, Christelle F-63400 Chamalieres (FR)**
• **LAURON, Bernard F-63110 Beaumont (FR)**

(74) Mandataire: **Noel, Chantal Odile et al Cabinet Orès 36, rue de St Pétersbourg 75008 Paris (FR)**

(56) Documents cités:
**WO-A-2007/041550     US-A1- 2006 189 475**

• **PICOZZI S ET AL: "Ozone adsorption on carbon nanotubes: Ab initio calculations and experiments" JOURNAL OF VACUUM SCIENCE AND TECHNOLOGY: PART A, AVS /AIP, MELVILLE, NY.; US, vol. 22, no. 4, 1 juillet 2004 (2004-07-01), pages 1466-1470, XP012073757 ISSN: 0734-2101**
• **VIRICELLE J P ET AL: "Selectivity improvement of semi-conducting gas sensors by selective filter for atmospheric pollutants detection" MATERIALS SCIENCE AND ENGINEERING C, ELSEVIER SCIENCE S.A, CH, vol. 26, no. 2-3, 1 mars 2006 (2006-03-01), pages 186-195, XP025137581 ISSN: 0928-4931 [extrait le 2006-03-01] cité dans la demande**

**EP 2 291 230 B1**

**Description**

L'invention concerne l'utilisation de nanomatériaux de carbone en tant que matériau de filtration perméable au dioxyde d'azote et imperméable à l'ozone. Elle concerne également un dispositif et un procédé de détection et/ou de quantification du dioxyde d'azote dans un mélange gazeux contenant de l'ozone et du dioxyde d'azote.

La qualité de l'air dans les villes industrialisées est une préoccupation mondiale.

Parmi tous les polluants, le dioxyde d'azote représente un problème majeur dans les zones urbaines. En effet, ce polluant est hautement concentré dans les centres villes et contribue à la formation d'un autre gaz toxique, l'ozone. Mais surtout le dioxyde d'azote a des effets très nocifs sur la santé humaine. A des concentrations aussi faibles que 15 ppb, des irritations nasales, des irritations oculaires et une toux sont associées à une exposition au dioxyde d'azote. A 30 ppb, une hyperactivité des muscles respiratoires se produit. Au-dessus de 80 ppb, on a reporté une incidence augmentée d'infections respiratoires et d'affections de la gorge.

Les directives données par l'Organisation Mondiale de la Santé pour une exposition au dioxyde d'azote sont de 100 ppb pendant une heure et de 75 ppb par jour. Dans un environnement urbain, ces valeurs peuvent être fortement dépassées. Ainsi, la quantité de dioxyde d'azote doit être contrôlée en continu.

Il a été proposé de détecter et de quantifier le dioxyde d'azote dans l'air ambiant en utilisant un microsystème capteur de gaz à base d'une couche mince de phtalocyanine de cuivre.

Ce système est décrit dans Brunet et al., Sensors & Actuators B130 (2008) 908-916.

Ce système comporte un capteur à couche mince de semi-conducteur résistif dans lequel le semi-conducteur résistif est la phtalocyanine de cuivre. La phtalocyanine de cuivre est quasi-insensible aux gaz réducteurs mais très sensible aux gaz oxydants tels que le dioxyde d'azote et l'ozone. Mais le système décrit dans ce document est un système embarqué dans les véhicules. Or dans les véhicules, l'ozone est inexistant et le dioxyde d'azote est le seul gaz polluant oxydant, ce qui permet au système décrit dans cet article de mesurer avec précision et sélectivité le dioxyde d'azote.

En effet, il est rappelé dans cet article que le capteur constitué d'une couche mince de phtalocyanine de cuivre ne permet pas le contrôle et la quantification sélective du dioxyde d'azote en présence d'ozone et que dans ce cas, il est nécessaire de filtrer l'ozone avant le passage de l'air sur le capteur à couches minces de phtalocyanine de cuivre.

Ainsi, Viricelle et al. dans « Selectivity improvement of semi-conducting gas sensors by selective filter for atmospheric pollutants detection », Materials Science and Engineering C, vol. 26, Issues 2-3, March 2006, 186-195, ont proposé un système de mesure sélective du dioxyde d'azote dans un mélange gazeux contenant du dioxyde d'azote et de l'ozone comprenant un capteur à couche mince de semi-conducteur qui est soit de l'oxyde d'étain, $SnO_2$, soit la phtalocyanine de cuivre, ce capteur comprenant, avant le capteur semi-conducteur un filtre constitué d'oxyde de manganèse, $MnO_2$, lorsque le semi-conducteur est l'oxyde d'étain, ou constitué d'indigo lorsque le semi-conducteur est la phtalocyanine de cuivre.

Cependant, avec le dispositif à semi-conducteur de $SnO_2$ et filtre de $MnO_2$, l'ozone était bien éliminé mais la sensibilité au dioxyde d'azote était également réduite. Le système à couche mince de semi-conducteur phtalocyanine de cuivre et à filtre rempli d'indigo présentait en revanche une grande sélectivité par rapport à $NO_2$.

Cependant, l'ozone réagit irréversiblement avec l'indigo, comme montré dans Viricelle et al., « Selectivity improvement of semi-conducting gas sensors by selective filter for atmospheric pollutants detection », Materials Science and Engineering C, Elsevier Science S.A, Ch, vol. 26, n°2-3, 1er mars 2006 (2006-03-01), page 186-195.

Ceci se traduit par une consommation du filtre dans le temps altérant sa durée de vie.

L'invention vise à pallier les inconvénients des systèmes de détection et/ou de quantification sélective du dioxyde d'azote dans un mélange gazeux contenant du dioxyde d'azote et de l'ozone, en proposant une structure de filtration présentant une interaction limitée à la surface d'un matériau actif et potentiellement régénérable.

A cet effet, l'invention propose l'utilisation de nanomatériaux de carbone ayant :

-   une surface spécifique, mesurée par BET, comprise entre 15 et 40 $m^2$/g inclus, et
-   un facteur de forme, égal au rapport (plus grande dimension/plus petite dimension) du nanomatériau, compris entre 5 et 250 inclus,

en tant que matériau de filtration, d'un mélange gazeux contenant du dioxyde d'azote et de l'ozone, perméable au dioxyde d'azote et imperméable à l'ozone.

De préférence les nanomatériaux ont une surface spécifique mesurée par BET, comprise entre 19 et 30 $m^2$/g inclus.

Dans un premier mode de mise en oeuvre préféré de l'utilisation de l'invention, les nanomatériaux comprennent 70% en poids de nanodisques et 30% en poids de nanocônes de carbone, ledit mélange ayant une surface spécifique, mesurée par BET, comprise entre 26 et 30 $m^2$/g inclus.

Dans un second mode de mise en oeuvre préféré de l'utilisation de l'invention, les nanomatériaux sont des nanofibres ayant une surface spécifique, mesurée par BET, de 19 m$^2$/g et un facteur de forme ayant un rapport égale (longueur/diamètre) de 250.

De préférence, le mélange gazeux est de l'air.

L'invention propose également un système de détection et/ou de quantification sélective du dioxyde d'azote dans un mélange gazeux contenant du dioxyde d'azote et de l'ozone, du type comprenant un capteur de gaz résistif à base d'une couche mince de semi-conducteur organique réactif au dioxyde d'azote et à l'ozone, caractérisé en ce qu'il comprend, en amont dudit capteur, un dispositif de filtration dudit mélange gazeux comprenant des particules de nanomatériaux de carbone ayant une surface spécifique, mesurée par BET, comprise entre 15 et 40 m$^2$/g inclus et un facteur de forme, égale au rapport (plus grande dimension/plus petite dimension) dudit nanomatériau, compris entre 5 et 250 inclus.

Par couche mince, on entend dans l'invention, une couche d'une épaisseur comprise entre 10 et 500 nanomètres, de préférence comprise entre 100 et 500 nanomètres.

De préférence, les nanomatériaux ont une surface spécifique comprise entre 19 et 30 m$^2$/g inclus.

Dans un premier mode de réalisation préféré du système de l'invention, les nanomatériaux sont des nanofibres ayant une surface spécifique de 19 m$^2$/g et un facteur de forme égal au rapport (longueur/diamètre) de 250.

Dans un second mode de réalisation préféré du système de l'invention, les nanomatériaux comprennent un mélange de 70 % en poids de nanodisques et de 30 % en poids de nanocônes, le mélange ayant une surface spécifique comprise entre 26 et 30m$^2$/g inclus.

Dans tous les modes de réalisation, dans le système de l'invention, de préférence le capteur est un capteur à base d'une couche mince, d'une épaisseur comprise entre 10 et 500 nanomètres, de préférence comprise entre 100 et 500 nanomètres, de phtalocyanine de cuivre.

Egalement, de préférence, le mélange gazeux est de l'air.

L'invention propose une méthode de détection et/ou de quantification sélective du dioxyde d'azote dans un mélange gazeux contenant du dioxyde d'azote et de l'ozone, du type comprenant le passage dudit mélange gazeux à travers un filtre puis du gaz filtré sur un capteur de gaz résistif à base d'une couche mince, d'une épaisseur comprise entre 10 et 500 nanomètres, de préférence comprise entre 100 et 500 nanomètres, de semi-conducteur organique réactif au dioxyde d'azote et à l'ozone, qui comprend le passage du gaz à travers un filtre contenant des nanomatériaux de carbone ayant une surface spécifique, mesurée par BET, comprise entre 15 et 40 m$^2$/g inclus et un facteur de forme, égal au rapport (plus grande dimension/plus petite dimension) des nanomatériaux de carbone, compris entre 5 et 250 inclus.

De préférence, dans la méthode de l'invention, les nanomatériaux ont une surface spécifique entre 19 et 30 m$^2$/g inclus.

Dans un premier mode de mise en oeuvre préféré de la méthode de l'invention, les nanomatériaux sont des nanofibres ayant une surface spécifique de 19 m$^2$/g et un facteur de forme, égal au rapport (diamètre/épaisseur) de 250.

Dans un second mode de mise en oeuvre préféré de la méthode de l'invention, les nanomatériaux comprennent un mélange de 70 % en poids de nanodisques et de 30 % en poids de nanocônes, le mélange ayant une surface spécifique comprise entre 26 et 30 m$^2$/g inclus.

L'invention sera mieux comprise, et d'autres avantages et caractéristiques de celle-ci apparaîtront plus clairement à la lecture de la description explicative qui suit.

L'invention repose sur la découverte que certains nanomatériaux de carbone ont la capacité de filtrer sélectivement l'ozone.

De plus, ces nanomatériaux sont régénérables, comme enseigné par M. Chesneau, dans le chapitre intitulé « Applications du carbone : les matériaux carbones activés », dans l'ouvrage : Le carbone dans tous ses états, Editeurs : P. Bernier et S. Lefrant, Gordon and Breach Science Publishers, 1997.

Au sens de l'invention, les termes « nanomatériaux de carbone » désignent des matériaux dont au moins une dimension est comprise entre 1 nanomètre et 350 nanomètres.

Les nanomatériaux peuvent être des nanoparticules, des nanofibres, des nanocônes, des nanodisques ou encore des nanotubes simples ou multiparois ou des mélanges de ceux-ci.

Les nanomatériaux de carbone sont des nanomatériaux en carbone ayant ou non une structure graphite.

Pour une utilisation en tant que matériau de filtration sélective de l'ozone, d'un mélange gazeux contenant du dioxyde d'azote et de l'ozone, les nanomatériaux de carbone pourront être soit des nanofibres, soit des nanocônes, soit des nanodisques, soit des nanotubes, soit encore des nanoparticules de carbone.

Des mélanges de tels nanomatériaux entrent également dans l'utilisation de l'invention.

Par « matériau de filtration sélectif de l'ozone », on entend, dans l'invention, un matériau de filtration qui est imperméable à l'ozone, c'est-à-dire qui retient l'ozone, et qui est perméable au dioxyde d'azote, c'est-à-dire qui laisse passer le dioxyde d'azote.

Ce sont les caractéristiques de surface spécifique et de facteur de forme des nanomatériaux de carbone utilisés dans l'invention qui en font un tel matériau de filtration sélectif de l'ozone.

Pour déterminer cette surface spécifique et ce rapport de forme, différents matériaux de carbone ont été testés.

Ces matériaux de carbone étaient d'une part des matériaux de carbone qui n'étaient pas des nanomatériaux, c'est-à-dire des particules de graphite ayant une taille moyenne, mesurée par granulométrie laser, de 4$\mu$m, et un échantillon de charbon actif MAXSORB, qui est un charbon actif commercialisé par Kansai Coke and Chemical Co, Japon, et d'autre part, des nanofibres, des nanotubes et des mélanges de nanodisques et de nanocônes de carbone.

Les nanofibres testées étaient :

- des nanofibres de carbone, notées dans ce qui suit CNF, obtenues par dépôt chimique en phase vapeur (CVD) puis traitées thermiquement à 1800°C, et fournies par la société MER Corporation, ayant une surface spécifique, mesurée par BET, de 19 m$^2$/g, et ayant un diamètre compris entre 80 et 350 nanomètres, et une longueur comprise entre 2 000 nanomètres et 20 000 nanomètres, c'est-à-dire un rapport de forme longueur/diamètre compris entre 5,7 et 250,0 et ayant une pureté de 90%, et

- ces mêmes nanofibres mais qui ont subi un broyage dans un broyeur à bille en acier inox pendant :

  • 20 mn sous air. Ces nanofibres sont notées CNFg20, dans ce qui suit, et avaient une surface spécifique, mesurée par BET, de 43 m$^2$/g,
  • 60 mn à l'air. Ces nanofibres sont notées dans ce qui suit CNFg60, et avaient une surface spécifique, mesurée par BET, de 87 m$^2$/g,
  • 12 h à l'air. Ces nanofibres sont notées dans ce qui suit CNF12h air et avaient une surface spécifique, mesurée par BET, de 505 m$^2$/g, et
  • 12 h sous argon. Ces nanofibres sont notées dans ce qui suit CNF12h Ar, et avaient une surface spécifique de 397 m$^2$/g.

Le broyage des nanofibres induit une rupture des fibres proportionnelle au temps du broyage. Après 12 h le caractère fibrillaire a disparu laissant la place à des grains de l'ordre du micron formé de l'agglomération de fibres rompues.

Les nanocônes et nanodisques de carbone testés étaient un mélange de 70% en masse de nanodisques, 20% en masse de nanocônes et 10% en masse de carbone amorphe, mélange fourni par N-TEC Norway, mélange dans lequel les nanodisques avaient un diamètres compris entre 800 nanomètres et 3 200 nanomètres et une épaisseur comprise entre 15 nanomètres et 65 nanomètres et les nanocônes avaient un diamètre compris entre 500 nanomètres et 2 800 nanomètres et une épaisseur comprise entre 15 et 65 nanomètres, ce qui correspond à un rapport de forme, pour les nanodisques, compris entre 12,3 et 213,3 et pour les nanocônes un rapport de forme compris entre 7,7 et 186,7 inclus.

Ce mélange de nanocônes, de nanodisques et de carbone amorphe avait une surface spécifique, mesurée par BET, de 30 m$^2$/g. Ce même mélange de nanocônes et nanodisques lavés a également été testé. Le lavage consistait en un lavage avec un excès de diméthylsulfoxyde pendant 30 mn dans un bain à ultrasons et avait pour but de solubiliser les résidus d'huiles lourdes non craquées déposés à la surface des nanodisques et nanocônes. Le carbone est séparé du solvant, qui prend une coloration jaune, par centrifugation à 10000 tours/min et enfin le solvant est évaporé à 150°C.

Après ce lavage, le mélange de nanocônes, noté CND lavés dans ce qui suit, avait une surface spécifique de 26 m$^2$/g.

Les nanotubes testés étaient des nanotubes simple paroi, notés dans la suite SWCNT, obtenus par la méthode de l'arc électrique, fourni par la société NANOLEDGE. Ces nanotubes monoparoi comportent un taux important de défauts. Ils contiennent 12% en masse de catalyseurs métalliques, 25% en masse de carbone amorphe et environ 10% de nanoparticules de structure graphite. Ces nanoparticules monoparoi avaient une surface spécifique de 165 m$^2$/g,

Le graphite testé était une poudre de graphite ayant une surface spécifique de 7 m$^2$/g. Cet échantillon est noté graphite dans le tableau qui suit.

Le charbon actif, noté MAXSORB dans ce qui suit, était constitué de particules de charbon d'un diamètre moyen de 1$\mu$m et avait une surface spécifique, mesurée par BET, de 3000 m$^2$/g.

Les conditions de tests étaient les suivantes : on a introduit des concentrations comprises entre 0 et 300 ppb d'ozone en mélange avec 0 à 300 ppb de dioxyde d'azote dans de l'air propre et asséché. Ces mélanges gazeux ont été introduits à un débit total de 45 litres/heure dans une chambre d'un volume de 0,88 cm$^3$ contenant 200 mg de chacun des matériaux de filtration cités ci-dessus. Le mélange gazeux filtré a ensuite été dosé quant au dioxyde d'azote et à l'ozone par deux analyseurs de gaz commerciaux, l'un analysant l'ozone et l'autre analysant le dioxyde d'azote.

Le pouvoir filtrant du matériau de filtration testé est noté $\eta$ dans ce qui suit et est égal à l'équation suivante :

$$\eta = 100 \times \left( \frac{C_{amont} - C_{aval}}{C_{amont}} \right)$$

On le voit d'après cette formule, lorsque l'ozone est retenue sur le matériau de filtration, $\eta$ tend vers 100 et lorsqu'elle n'est pas retenue par le matériau et donc traverse le matériau de filtration c'est-à-dire ne joue pas son rôle de matériau de filtration sélectif pour l'ozone, $\eta$ de l'ozone tend vers 0.

Le matériau de filtration de l'invention doit être imperméable à $O_3$ mais perméable à $NO_2$. Pour cette raison le $\eta$ de $NO_2$ doit tendre vers 0.

Autrement dit, le matériau de filtration totalement sélectif à l'ozone et totalement perméable à $NO_2$ doit avoir un $\eta$ de l'ozone égal à 100 et un $\eta$ de $NO_2$ égal à 0.

Les résultats obtenus sont regroupés dans le tableau 1 suivant :

Tableau 1

| Matériau filtrant | Témoin MAXSORB | Témoin Graphite | CNFg20 | CNFg60 | CNF12h air | CNF12h Ar | CNF | CND | CND lavé | SWCNT |
|---|---|---|---|---|---|---|---|---|---|---|
| $SS(m^2/g)$ | 1 500 | 7 | 43 | 87 | 505 | 397 | 19 | 30 | 26 | 165 |
| $\eta O_3$ | 91,0 | 29,1 | 93,9 | 95,9 | 97,5 | 97,4 | 91,6 | 98,7 | 96,7 | 98,6 |
| $\eta NO_2$ | 91,0 | 49,6 | 36,2 | 71,6 | 32,3 | 50,4 | ~0 | 5,0 | 3,2 | 54,2 |

On voit d'après le tableau 1 que les échantillons CNF, CND et CND lavé, qui ont une surface spécifique mesurée par BET comprise entre 19 et 30 m$^2$/g inclus, retiennent plus de 90% de l'ozone tout en retenant moins de 5% du dioxyde d'azote. En revanche, lorsque la surface spécifique est de 43 m$^2$/g, comme cela est le cas pour l'échantillon noté CNFg20, le pouvoir filtrant de ces nanofibres pour l'ozone est de 93,9, c'est-à-dire supérieur au pouvoir filtrant de l'ozone de l'échantillon noté CNF, mais le matériau CNFg20 retient également 36% du dioxyde d'azote, ce qui en fait un matériau non adapté.

Pour ces raisons, les nanomatériaux utilisés en tant que matériau de filtration sélectif de l'ozone ont une surface spécifique, mesurée par BET comprise entre 15 et 40 m$^2$/g inclus, et de préférence compris entre 19 et 30 m$^2$/g inclus.

Mais, la surface spécifique du matériau n'est pas le seul facteur du pouvoir filtrant du nanomatériau de carbone. Le facteur de forme est également important, car c'est ce qui permet au matériau de filtration d'exercer pleinement une action filtrante.

Ainsi, dans un mode de réalisation préféré, les nanomatériaux de carbone utilisés en tant que matériau de filtration sélectif de $O_3$ comprend des nanofibres de carbone ayant une surface spécifique de 19 m$^2$/g et un facteur de forme, égal au rapport longueur/diamètre, compris entre 5 et 250 inclus, et plus préférablement de 250.

Dans un autre mode de réalisation préféré de l'invention, le matériau de filtration sélectif de $O_3$ utilisé dans l'invention est composé d'un mélange de 70% en poids de nanodisques, 20% en poids de nanocônes et 10% en poids de carbone amorphe lavé ou non lavé, ayant une surface spécifique comprise entre 26 et 30 m$^2$/g, les nanodisques ayant un rapport de forme compris entre 12,3 et 213,3 inclus, de préférence de 213,3 et les nanocônes ayant un facteur de forme compris entre 7,7 et 186,7, de préférence égal 186,7.

Dans un mode de réalisation préféré de l'invention, le matériau de filtration de l'invention est utilisé en tant que matériau de remplissage d'un filtre placé en amont d'un capteur de gaz résistif à base d'une couche mince de semi-conducteur organique réactif au dioxyde d'azote et à l'ozone, pour former un système de détection et/ou de quantification sélective du dioxyde d'azote dans un mélange gazeux contenant du dioxyde d'azote et de l'ozone.

Un capteur résistif à base de couches minces de semi-conducteur adapté est en particulier un capteur à base de couches minces de phtalocyanine de cuivre.

En effet, la phtalocyanine de cuivre est quasi insensible aux gaz réducteurs et très sensible aux gaz oxydants et en particulier à l'ozone et au dioxyde d'azote. En filtrant, sélectivement l'ozone, grâce au dispositif de filtration contenant le matériau de filtration de l'invention, du mélange gazeux, avant son passage sur ce capteur, on éliminera l'ozone et seul le dioxyde d'azote sera détecté et quantifié.

Le mélange gazeux est plus particulièrement de l'air.

Ainsi, une méthode de détection et/ou de quantification sélective du dioxyde d'azote dans un mélange gazeux, et en particulier de l'air, contenant du dioxyde d'azote et de l'$O_3$ comprend une étape de passage dudit mélange gazeux à travers un filtre contenant des nanomatériaux de carbone selon l'invention.

Les nanomatériaux de l'invention pourront être également utilisés dans tous systèmes et dans toutes méthodes de détection et/ou de quantification sélective du dioxyde d'azote, éventuellement avec un autre capteur qu'un capteur à base de couche mince de phtalocyanine de cuivre, comme cela apparaîtra à l'homme de l'art.

De plus, les nanomatériaux de l'invention peuvent être fonctionnalisés pour augmenter leur pouvoir de filtration sélectif de l'ozone par rapport au dioxyde d'azote, par exemple en les fonctionnalisant avec des matériaux très sélectifs vis-à-vis de l'ozone, tels que par exemple l'indigo, ou par des fonctions styrène ou acrylonitrile, en créant une liaison chimique entre la nanomatériau et le groupement de fonctionnalisation.

D'une manière générale, le groupement de fonctionnalisation est un groupement organique ayant au moins une double liaison pour le greffage sur le carbone du nanomatériau de carbone et au moins une autre liaison, double ou triple, insaturée pour augmenter le pouvoir de filtration sélectif de l'ozone des matériaux de carbone.

La fonctionnalisation avec l'indigo pourra également être appliquée par greffage physique. Dans ce cas, les interactions entre la molécule d'indigo et la surface de nanocarbone sont de type Van der Waal (pi-stacking).

Plus précisément, une solution d'acétonitrile saturée en indigo est mise en contact avec la poudre de nanocarbone (CND traités à 2 600°C pour accroître le degré de graphitation, CNF) puis le mélange est sonifié pendant 20 minutes. Après décoloration de la solution et décantation du nanocarbone, le liquide surnageant est éliminé. La poudre obtenue est placée sous vide primaire à 100°C pendant 12 heures pour éliminer les traces de solvant. La décoloration de la solution est quantifiée par spectrométrie UV-Visible (Lambda = 600 nm : longueur maximale d'absorbance de l'indigo).

Cette fonctionnalisation par pi-stacking est privilégiée pour les nanocarbones post-traités à haute température pour accroître leur taux de graphitation (1 800 et 2600°C pour les nanofibres et les mélanges de nanocônes et nanodisques, respectivement). Le pouvoir absorbant des mélanges de nanocônes et des nanodisques ayant subi un traitement thermique à 2 600°C est de 52 mg d'indigo par gramme de carbone.

**EP 2 291 230 B1**

**Revendications**

1. Utilisation de nanomatériaux de carbone ayant :

- une surface spécifique, mesurée par BET, comprise entre 15 et 40 m²/g inclus, et
- un facteur de forme, égal au rapport (plus grande dimension/plus petite dimension) du nanomatériau, compris entre 5 et 250 inclus,
en tant que matériau de filtration, d'un mélange gazeux contenant du dioxyde d'azote et de l'ozone, perméable au dioxyde d'azote et imperméable à l'ozone.

2. Utilisation selon la revendication 1, **caractérisée en ce que** les nanomatériaux ont une surface spécifique comprise entre 19 et 30 m²/g inclus.

3. Utilisation selon la revendication 1 ou 2, **caractérisée en ce que** les nanomatériaux comprennent 70% en poids de nanodisques et 30% en poids de nanocônes de carbone, ledit mélange ayant une surface spécifique, mesurée par BET, comprise entre 26 et 30 m²/g inclus.

4. Utilisation selon la revendication 1 ou 2 **caractérisée en ce que** les nanomatériaux sont des nanofibres de carbone ayant une surface spécifique, mesurée par BET, de 19 m²/g et un facteur de forme, égal au rapport (longueur/diamètre) de 250.

5. Utilisation selon l'une quelconque des revendications précédentes **caractérisée en ce que** le mélange gazeux est de l'air.

6. Système de détection et/ou de quantification sélective du dioxyde d'azote dans un mélange gazeux contenant du dioxyde d'azote et de l'ozone, du type comprenant un capteur de gaz résistif à base d'une couche mince ayant une épaisseur comprise entre 10 et 500 nm, de préférence entre 100 et 500 nm de semi-conducteur organique réactif au dioxyde d'azote et à l'ozone, **caractérisé en ce qu'**il comprend, en amont dudit capteur, un dispositif de filtration dudit mélange gazeux comprenant des nanomatériaux de carbone ayant une surface spécifique, mesurée par BET, comprise entre 15 et 40 m²/g inclus et un facteur de forme, égal au rapport (plus grande dimension/plus petite dimension) dudit nanomatériau, compris entre 5 et 250 inclus.

7. Système selon la revendication 6, **caractérisé en ce que** les nanomatériaux ont une surface spécifique comprise entre 19 et 30 m²/g inclus.

8. Système selon la revendication 6 ou 7, **caractérisé en ce que** les nanomatériaux sont des nanofibres ayant une surface spécifique de 19 m²/g et un facteur de forme égal au rapport (longueur/diamètre) égal à 250.

9. Système selon la revendication 6 ou 7, **caractérisé en ce que** les nanomatériaux comprennent un mélange de 70 % en poids de nanodisques et 30 % en poids de nanocônes, le mélange ayant une surface spécifique comprise entre 26 et 30m²/g inclus.

10. Système selon l'une quelconque des revendications 6 à 9, **caractérisé en ce que** ledit capteur est un capteur à base d'une couche mince, ayant une épaisseur comprise entre 10 et 500 nm, de préférence entre 100 et 500 nm, de phtalocyanine de cuivre.

11. Système selon l'une quelconque des revendications 6 à 10, **caractérisé en ce que** le mélange gazeux est de l'air.

12. Méthode de détection et/ou de quantification sélective du dioxyde d'azote dans un mélange gazeux contenant du dioxyde d'azote et de l'ozone du type comprenant le passage dudit mélange gazeux à travers un filtre puis du gaz filtré sur un capteur de gaz résistif à base d'une couche mince, ayant une épaisseur comprise entre 10 et 500 nm, de préférence entre 100 et 500 nm, de semi-conducteur organique réactif au dioxyde d'azote et à l'ozone, **caractérisée en ce qu'**elle comprend le passage du mélange gazeux à travers un filtre contenant des nanomatériaux de carbone ayant une surface spécifique, mesurée par BET, comprise entre 15 et 40 m²/g inclus et un facteur de forme, égal au rapport (plus grande dimension/plus petite dimension) compris entre 5 et 250 inclus.

13. Méthode la revendication 12, **caractérisée en ce que** les nanomatériaux ont une surface spécifique entre 19 et 30 m²/g inclus.

**14.** Méthode selon la revendication 12 ou 13, **caractérisée en ce que** les nanomatériaux sont des nanofibres ayant une surface spécifique de 19 m$^2$/g et un facteur de forme égal au rapport (longueur/diamètre) égal à 250.

**15.** Méthode selon la revendication 12 ou 13, **caractérisée en ce que** les nanomatériaux comprennent un mélange de 70 % en poids de nanodisques et de 30 % en poids de nanocônes, le mélange ayant une surface spécifique comprise entre 26 et 30 m$^2$/g inclus.

**Patentansprüche**

**1.** Verwendung von Kohlenstoffnanomaterialien, die

- eine spezifische Oberfläche, gemessen nach BET, zwischen einschließlich 15 und 40 m$^2$/g und
- einen Formfaktor gleich dem Verhältnis (größte Dimension/kleinste Dimension) des Nanomaterials zwischen einschließlich 5 und 250 haben,
als Filtermaterial, das durchlässig für Stickstoffdioxid und undurchlässig für Ozon ist, für eine Gasmischung, die Stickstoffdioxid und Ozon enthält.

**2.** Verwendung nach Anspruch 1, **dadurch gekennzeichnet, dass** die Nanomaterialien eine spezifische Oberfläche zwischen einschließlich 19 und 30 m$^2$/g haben.

**3.** Verwendung nach Anspruch 1 oder 2, **dadurch gekennzeichnet, dass** die Nanomaterialien 70 Gew.-% Kohlenstoffnanodiscs und 30 Gew.-% Kohlenstoffnanokegel umfassen, wobei die Mischung eine spezifische Oberfläche, gemessen nach BET, zwischen einschließlich 26 und 30 m$^2$/g hat.

**4.** Verwendung nach Anspruch 1 oder 2, **dadurch gekennzeichnet, dass** die Nanomaterialien Kohlenstoffnanofasern sind, die eine spezifische Oberfläche, gemessen nach BET, von 19 m$^2$/g und einen Formfaktor gleich dem Verhältnis (Länge/Durchmesser) von 250 haben.

**5.** Verwendung nach einem der vorhergehenden Ansprüche, **dadurch gekennzeichnet, dass** die Gasmischung Luft ist.

**6.** System zum selektiven Nachweis und/oder zur selektiven Quantifizierung von Stickstoffdioxid in einer Gasmischung, die Stickstoffdioxid und Ozon enthält, umfassend einen resistiven Gassensor auf Basis einer organischen Halbleiterdünnschicht mit einer Dicke zwischen 10 und 500 nm, vorzugsweise zwischen 100 und 500 nm, der mit dem Stickstoffdioxid und dem Ozon reagiert, **dadurch gekennzeichnet, dass** es, dem Sensor vorgeschaltet, eine Filtriervorrichtung für die Gasmischung umfasst, die Kohlenstoffnanomaterialien umfasst, die eine spezifische Oberfläche, gemessen nach BET, zwischen einschließlich 15 und 40 m$^2$/g und einen Formfaktor gleich dem Verhältnis (größte Dimension/kleinste Dimension) des Nanomaterials zwischen einschließlich 5 und 250 haben.

**7.** System nach Anspruch 6, **dadurch gekennzeichnet, dass** die Nanomaterialien eine spezifische Oberfläche zwischen einschließlich 19 und 30 m$^2$/g haben.

**8.** System nach Anspruch 6 oder 7, **dadurch gekennzeichnet, dass** die Nanomaterialien Nanofasern sind, die eine spezifische Oberfläche von 19 m$^2$/g und einem Formfaktor gleich dem Verhältnis (Länge/Durchmesser) von 250 haben.

**9.** System nach Anspruch 6 oder 7, **dadurch gekennzeichnet, dass** die Nanomaterialien eine Mischung aus 70 Gew.-% Nanodiscs und 30 Gew.-% Nanokegeln umfassen, wobei die Mischung eine spezifische Oberfläche zwischen einschließlich 26 und 30 m$^2$/g hat.

**10.** System nach einem der Ansprüche 6 bis 9, **dadurch gekennzeichnet, dass** der Sensor ein Sensor auf Basis einer Kupferphthalocyanindünnschicht mit einer Dicke zwischen 10 und 500 nm, vorzugsweise zwischen 100 und 500 nm ist.

**11.** System nach einem der Ansprüche 6 bis 10, **dadurch gekennzeichnet, dass** die Gasmischung Luft ist.

**12.** Verfahren zum selektiven Nachweis und/oder zur selektiven Quantifizierung von Stickstoffdioxid in einer Gasmischung, die Stickstoffdioxid und Ozon enthält, umfassend Strömenlassen der Gasmischung durch ein Filter und anschließend des filtrierten Gases über einen resistiven Gassensor auf Basis einer organischen Halbleiterdünnschicht mit einer Dicke zwischen 10 und 500 nm, vorzugsweise zwischen 100 und 500 nm, der mit dem Stickstoffdioxid und dem Ozon reagiert, **dadurch gekennzeichnet, dass** es Strömenlassen der Gasmischung durch ein Filter umfasst, das Kohlenstoffnanomaterialien enthält, die eine spezifische Oberfläche, gemessen nach BET, zwischen einschließlich 15 und 40 $m^2$/g und einen Formfaktor gleich dem Verhältnis (größte Dimension/kleinste Dimension) zwischen einschließlich 5 und 250 haben.

13. Verfahren nach Anspruch 12, **dadurch gekennzeichnet, dass** die Nanomaterialien eine spezifische Oberfläche zwischen einschließlich 19 und 30 $m^2$/g haben.

14. Verfahren nach Anspruch 12 oder 13, **dadurch gekennzeichnet, dass** die Nanomaterialien Nanofasern sind, die eine spezifische Oberfläche von 19 $m^2$/g und einem Formfaktor gleich dem Verhältnis (Länge/Durchmesser) von 250 haben.

15. Verfahren nach Anspruch 12 oder 13, **dadurch gekennzeichnet, dass** die Nanomaterialien eine Mischung aus 70 Gew.-% Nanodiscs und 30 Gew.-% Nanokegel umfassen, wobei die Mischung eine spezifische Oberfläche zwischen einschließlich 26 und 30 $m^2$/g hat.

**Claims**

1. Use of carbon nanomaterials having:

   - a specific surface area, measured by BET, of between 15 and 40 $m^2$/g inclusive, and
   - a form factor, equal to the ratio (larger dimension/smaller dimension) of the nanomaterial, of between 5 and 250 inclusive,
   as a material for filtering a gaseous mixture containing nitrogen dioxide and ozone which is permeable to nitrogen dioxide and imperméable to ozone.

2. Use according to claim 1, **characterized in that** the nanomaterials have a specific surface area of between 19 and 30 $m^2$/g inclusive.

3. Use according to claim 1 or 2, **characterized in that** the nanomaterials comprise 70% by weight of nanodises and 30% by weight of carbon nanocones, said mixture having a specific surface area, measured by BET, of between 26 and 30 $m^2$/g inclusive.

4. Use according to claim 1 or 2, **characterized in that** the nanomaterials are carbon nanofibres having a specific surface area, measured by BET, of 19 $m^2$/g and a form factor equal to the ratio (length/diameter) of 250.

5. Use according to any of the preceding claims, **characterized in that** the gaseous mixture is air.

6. System for detection and/or selective quantification of nitrogen dioxide in a gaseous mixture containing nitrogen dioxide and ozone, of the type comprising a resistive gas sensor based on a thin layer having a thickness of between 10 and 500 nm, preferably between 100 and 500 nm of organic semiconductor reactive to nitrogen dioxide and ozone, **characterized in that** it comprises, upstream of said sensor, a device for filtering said gaseous mixture comprising carbon nanomaterials having a specific surface area, measured by BET, of between 15 and 40 $m^2$/g inclusive and a form factor, equal to the ratio (larger dimension/smaller dimension) of said nanomaterial, of between 5 and 250 inclusive.

7. System according to claim 6, **characterized in that** the nanomaterials have a specific surface area of between 19 and 30 $m^2$/g inclusive.

8. System according to claim 6 or 7, **characterized in that** the nanomaterials are carbon nanofibres having a specific surface area of 19 $m^2$/g and a form factor equal to the ratio (length/diameter) equal to 250.

9. System according to claim 6 or 7, **characterized in that** the nanomaterials comprise a mixture of 70% by weight of nanodiscs and 30% by weight of nanocones, the mixture having a specific surface area of between 26 and 30

$m^2/g$ inclusive.

10. System according to any of claims 6 to 9, **characterized in that** said sensor is a sensor based on a thin layer, having a thickness of between 10 and 500 nm, preferably between 100 and 500 nm, of copper phthalocyanine.

11. System according to any of claims 6 to 10, **characterized in that** the gaseous mixture is air.

12. Method for detection and/or selective quantification of nitrogen dioxide in a gaseous mixture containing nitrogen dioxide and ozone of the type comprising the passing of said gaseous mixture through a filter then the filtered gas over a resistive gas sensor based on a thin layer, having a thickness of between 10 and 500 nm, preferably between 100 and 500 nm, of organic semiconductor reactive to nitrogen dioxide and ozone, **characterized in that** it comprises the passing of the gaseous mixture through a filter containing carbon nanomaterials having a specific surface area, measured by BET, of between 15 and 40 $m^2/g$ inclusive and a form factor, equal to the ratio (larger dimension/smaller dimension) of between 5 and 250 inclusive.

13. Method according to claim 12, **characterized in that** the nanomaterials have a specific surface area of between 19 and 30 $m^2/g$ inclusive.

14. Method according to claim 12 or 13, **characterized in that** the nanomaterials are nanofibres having a specific surface area of 19 $m^2/g$ and a form factor equal to the ratio (length/diameter) equal to 250.

15. Method according to claim 12 or 13, **characterized in that** the nanomaterials comprise a mixture of 70% by weight of nanodiscs and 30% by weight of nanocones, the mixture having a specific surface area of between 26 and 30 $m^2/g$ inclusive.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BRUNET et al.** *Sensors & Actuators,* 2008, vol. B130, 908-916
- **VIRICELLE et al.** Selectivity improvement of semi-conducting gas sensors by selective filter for atmospheric polluants detection. *Materials Science and Engineering C,* Mars 2006, vol. 26 (2-3), 186-195
- Selectivity improvement of semi-conducting gas sensors by selective filter for atmospheric polluants detection. **VIRICELLE et al.** Materials Science and Engineering C. Elsevier Science S.A, 01 Mars 2006, vol. 26, 186-195
- Applications du carbone : les matériaux carbones activés. **M. CHESNEAU.** Le carbone dans tous ses états. Gordon and Breach Science Publishers, 1997